(19) Europäisches Patentamt
European Patent Office
Office européen des brevets

(11) Publication number: 0 143 440 A2

(12) **EUROPEAN PATENT APPLICATION**

(21) Application number: 84114137.7

(22) Date of filing: 22.11.84

(51) Int. Cl.⁴: C 07 D 271/10, A 01 N 43/82 // C07C109/04, C07C133/00

(30) Priority: 28.11.83 JP 223771/83

(43) Date of publication of application: 05.06.85 Bulletin 85/23

(84) Designated Contracting States: DE FR GB IT

(71) Applicant: MITSUBISHI CHEMICAL INDUSTRIES LIMITED, 5-2, Marunouchi 2-chome Chiyoda-ku, Tokyo 100 (JP)

(72) Inventor: Yamamoto, Izuru Mitsubishi Chem. Industries Ltd, Sogo Kenkyusho No. 1000, Kamoshida-cho, Midori-ku Yokohama-shi Kanagawa-ken (JP)
Inventor: Kyomura, Nobuo Mitsubishi Chem. Industries Ltd, Sogo Kenkyusho No. 1000, Kamoshida-cho, Midori-ku Yokohama-shi Kanagawa-ken (JP)
Inventor: Satoh, Katsutoshi Mitsubishi Chem. Industries Ltd, Sogo Kenkyusho No. 1000, Kamoshida-cho, Midori-ku Yokohama-shi Kanagawa-ken (JP)
Inventor: Takahashi, Yohji Mitsubishi Chem. Industries Ltd, Sogo Kenkyusho No. 1000, kamoshida-cho, Midori-ku Yokohama-shi Kanagawa-ken (JP)

(74) Representative: Wächtershäuser, Günter, Dr., Tal 29, D-8000 München 2 (DE)

(54) **Insecticidal composition.**

(57) An insecticidal composition comprising an insecticidally effective amount of at least one oxadiazolinone derivative represented by the general formula:

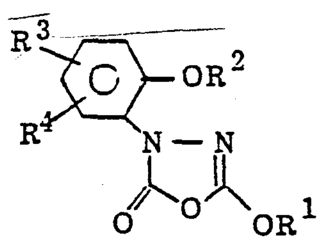

where $R^1$ is an alkyl group having from 1 to 5 carbon atoms, $R^2$ is an alkyl group having from 1 to 5 carbon atoms, and an alkenyl group having from 2 to 5 carbon atoms or an alkynyl group having from 2 to 5 carbon atoms, and each of $R^3$ and $R^4$ is a hydrogen atom, an alkyl group having from 1 to 4 carbon atoms or a trifluoromethyl group, provided that $R^3$ and $R^4$ are not simultaneously hydrogen atoms, and an adjuvant.

EP 0 143 440 A2

INSECTICIDAL COMPOSITION

The present invention relates to an insecticidal composition. More particularly, it relates to an insecticidal composition containing a 1,3,4-oxadiazolinone derivative as an active ingredient.

Japanese Unexamined Patent Publication No. 58140/1973 discloses insecticidal and acaricidal agents containing, as an active ingredient, a 5-(alkoxy or phenoxy)-3-phenyl-1,3,4-oxadiazoline-2-one with its phenyl group unsubstituted or substituted by one or two methyl groups or halogen atoms. Japanese Unexamined Patent Publication No. 101981/1976 (which corresponds to French Patent No. 2,299,328 and U.S. Patent No. 4,150,142) discloses that 5-alkoxy-3-substituted phenyl-1,3,4-oxadiazoline-2-ones having only one substituent at the 2-position of the phenyl group, have insecticidal, acaricidal and nematocidal activities, and that such compounds have higher insecticidal effectiveness than the compounds disclosed in the above-mentioned Japanese Unexamined Patent Publication No. 58140/1973. Further, Japanese

Unexamined Patent Publication No. 57703/1971 (which corresponds to French Patent No. 2,466,194) discloses a method for controlling green rice leafhoppers by means of the compounds disclosed in Japanese Unexamined Patent Publication No. 101981/1976.

In general, even a minor modification in the structure of a chemical substance, such as a change in the type, number or positions of substituents, substantially affect the physiological properties of the substance. Accordingly, it is hardly possible to predict or foresee any insecticidal activity of a compound merely from the similarity in the chemical structure.

German Patent No. 2604110 discloses that oxadiazolinone derivatives having an alkoxy group at the 2-position on the phenyl group and further substituents such as alkyl groups or halogens, are effective as anthelminthics for the control of parasites on sheep, horses or dogs. However, such parasites in the animal bodies are nematodes. It is difficult to foresee insecticidal activities from the activity against such nematodes.

The present inventors have conducted extensive researches on the substituents on the phenyl group of a 3-substituted phenyl oxadiazolinone derivative with an aim to develop an insecticide having a superior insecticidal activity, and have finally found that 5-alkoxy-3-substituted phenyl-1,3,4-oxadiazoline-2-ones

having at least two substituents on the phenyl group, one of the substituents being an alkoxy group at the 2-position, have excellent insecticidal activities.

Thus, the present invention provides an insecticidal composition comprising an insecticidally effective amount of at least one oxadiazolinone derivative represented by the general formula:

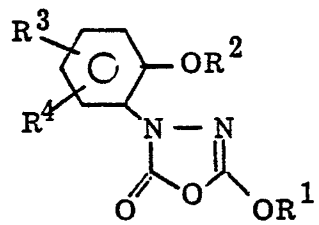

(I)

where $R^1$ is an alkyl group having from 1 to 5 carbon atoms, $R^2$ is an alkyl group having from 1 to 5 carbon atoms, and an alkenyl group having from 2 to 5 carbon atoms or an alkynyl group having from 2 to 5 carbon atoms, and each of $R^3$ and $R^4$ is a hydrogen atom, an alkyl group having from 1 to 4 carbon atoms or a trifluoromethyl group, provided that $R^3$ and $R^4$ are not simultaneously hydrogen atoms, and an adjuvant.

Now, the present invention will be described in detail with reference to the preferred embodiments.

As $R^1$ and $R^2$ in the oxadiazolinone derivative represented by the above formula I, there may be mentioned a straight chain or branched alkyl group such as methyl, ethyl, n-propyl, i-propyl, sec-butyl, tert-butyl, n-pentyl or sec-pentyl. Further, $R^2$ includes an

0143440

alkenyl group such as allyl or an alkynyl group such as propargyl. As $R^3$ and $R^4$, there may be mentioned an alkyl group such methyl, ethyl, n-propyl, i-propyl or sec-butyl, a trifluoromethyl group or a hydrogen atom.

Particularly preferred as an insecticide is the one wherein $R^1$ is methyl or ethyl. $R^2$ is also preferably methyl or ethyl. Further, it is preferred that one of $R^3$ and $R^4$ is methyl and the other is hydrogen, and more preferably, the methyl group is substituted at the 4- or 5-position, particularly at the 4-position, of the phenyl group. Specifically, there may be mentioned 3-(2-ethoxy-5-methylphenyl)-5-methoxy-1,3,4-oxadiazoline-2-one, 3-(2-ethoxy-4-methylphenyl)-5-methoxy-1,3-4-oxadiazoline-2-one and 3-(2-methoxy-4-methylphenyl)-5-methoxy-1,3,4-oxa-diazoline-2-one.

Among the compounds of the formula I, 5-methoxy-3-(2-methoxy-4-methylphenyl)-1,3,4-oxadiazoline-2-one is disclosed as an active ingredient of an anthelminthic for animals in German Patent No. 2604110, as mentioned above.

The compounds of the formula I may be prepared, for instance, by the following reactions.

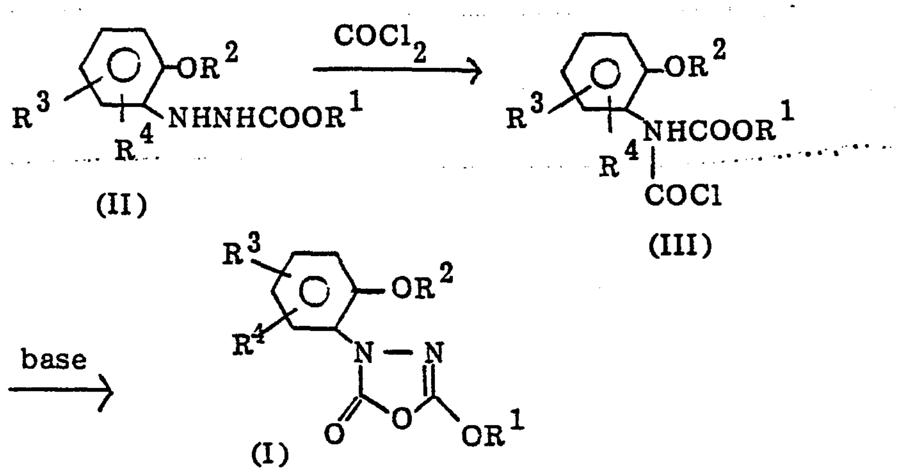


(II) (III) (I)

In the above reaction formulas, $R^1$, $R^2$, $R^3$ and $R^4$ are as defined above.

The first reaction is conducted in a solvent such as benzene, toluene, methylene chloride, chloroform, carbon tetrachloride, tetrahydrofuran or pyridine, in the presence of phosgene, at a temperature of from -20 to 150°C. The subsequent cyclization is conducted in the presence of a base such as pyridine, triethylamine or N,N-diethylaniline at a temperature of from -20 to 150°C.

In the above reaction, the carbazinates of the formula II used as the starting material, may be prepared by reacting a 2-alkoxyphenylhydrazine represented by the general formula:

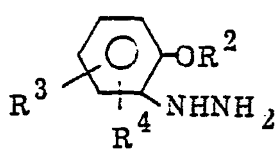

(IV)

where $R^2$, $R^3$ and $R^4$ are as defined above, with a chloroformate in a solvent such as benzene, toluene, dichloromethane, chloroform, carbon tetrachloride or

tetrahydrofuran, in the presence of a base such as pyridine, triethylamine, N,N-dimethylaniline or sodium hydroxide.

The compounds of the formula I exhibit remarkable insecticidal activities, particularly when contacted to or took in by insects. They have remarkable insecticidal activities against such insects as Hemiptera such as green rice leafhoppers(Nephotettix cincticeps Uhler), brown plant hoppers(Nilaparvata lugens Stal), white-backed rice plant hoppers(Sogota furcifera Horvth), aphids, greenhouse white flies or bugs; Orthoptera such as american cockroach(Periplaneta americana Linne), smoky brown cockroach or german cockroach(Blattera germanica Linne); or Coleoptera such as adzuki bean weevil (Callosobruchus chinensis Linne). They are particularly effective against green rice leafhoppers.

When the compounds of the formula I are to be used as insecticides, they may be used by themselves. However, they are usually formulated into an emulsion, a dust, a wettable powder, a granule or an emulsifiable concentrate, with use of adjuvants as in the case of conventional agricultural chemicals, and then used as such or after being diluted. As the adjuvants, those commonly used in the conventional insecticidal formulations may be employed. For instance, there may be mentioned a solid carrier such as talc, kaolin, diatomaceous earth, clay or starch; a solvent such as water, xylene, toluene, dimethylformamide or

acetonitrile; or other known surfactants such as an emulsifying agent or a dispersing agent.

The proportion of each ingredient, particularly the concentration of the compound of the formula I as the active ingredient in a formulated insecticidal composition is not limited to any particular range, and may vary depending upon the type of the particular formulation. However, the concentration of the active ingredient is preferably from 20 to 95% by weight in the case of a wettable powder, from 0.5 to 10% by weight in the case of a dust, from 0.5 to 25% by weight in the case of a granule, or from 10 to 50% by weight in the case of an emulsifiable concentrate.

Further, the compound of the formula I may be combined with other insecticide, an acaricide, a bactericide or a herbicide in an optional ratio. Particularly when the compound of the formula I is combined with another insecticide such as carbamate, organophosphate or pyrethroid, the respective activities are expected to be stabilized and help each other to provide a synergism.

As organophosphorus insecticides which may be incorporated, there may be mentioned organophosphorus insecticides having a dialkylthio group or a dialkyloxy group, such as S,S,S-tributyl phosphorotrithioate (DEF), S-benzyl O,O-diisopropyl phosphorothioate (IBP), 4-(methylthio)phenyl dipropylphosphate (Propahos), O-ethyl S,S-dipropyl phosphorodithioate, 4-(nitro)phenyl dipropyl

phosphate, S,S-di-n-propyl N-n-propyl phosphoroamidodithioate, S,S-di-n-butyl N-n-propyl phosphoroamidodithioate, S,S-di-n-propyl N-n-propyl phosphoroamidotrithioate, S,S-di-n-butyl N-n-propyl phosphoroamidotrithioate, O,O-di-n-propyl N-n-propyl phosphoroamidate and O,O-di-n-butyl N-propyl phosphoroamidothioate.

As carbamate insecticides, there may be mentioned N-methylcarbamates such as 2-isopropylphenyl N-methylcarbamate (MIPC), 2-sec-butylphenyl N-methylcarbamate (BPMC), m-tolyl N-methylcarbamate (MTMC), 3,4-xylyl N-methylcarbamate (MPMC), 3,5-xylyl N-methylcarbamate (XMC), 2-isopropoxyphenyl N-methylcarbamate (PHC) and 1-naphthyl N-methylcarbamate (NAC). The ratio of the carbamate to the compound of the formula I may be selected within a wide range, but is usually from 0.1 to 10 parts by weight of the carbamate relative to 1 part by weight of the compound of the formula I.

The amount of the application of the insecticidal composition of the present invention varies depending upon the type of the insect to be controlled and the season for the application and is selected within a wide range. For instance, for the control of green rice leafhoppers, a dust containing from 0.5 to 5% by weight, preferably from 2 to 3% by weight of the active ingredient is applied in an amount of from 2 to 4 kg per 10 ares.

Now, the present invention will be described in further detail by Examples with respect to the process for the preparation of the compounds, the formulations and the insecticidal activities. However, it should be understood that the present invention is by no means restricted by such specific Examples.

EXAMPLE 1:

A suspension comprising 6.85 g (0.05 mol) of 5-methyl-O-anisidine and 150 ml of concentrated hydrochloric acid, was cooled to 0°C, and a solution of 3.86 g (0.056 mol) of sodium nitrite in 20 ml of water, was dropwise added thereto over a period of 15 minutes.

Then, the mixture was stirred at from 5 to 10°C for 1.5 hours. After decomposing excess nitrous acid with sulfamic acid, a solution of 33.8 g (0.15 mol) of stannous chloride dihydrate in 35 ml of concentrated hydrochloric acid was added while maintaining the reaction mixture at a temperature of from 0 to 5°C. The reaction mixture was cooled to room temperature, and the precipitated crystals were collected by filtration, then dissolved in 160 ml of ice water, adjusted to pH 11 with a 5% sodium hydroxide aqueous solution and extracted with dichloromethane. The organic layer thereby obtained was dried over anhydrous sodium sulfate, and the solvent was distilled off, whereby 6.08 g (yield: 80%) of 5-methyl-2-methoxy-phenylhydrazine was obtained as a yellow oily substance. The chemical structure was ascertained by IR

and NMR. Then, the product was used for the following reaction without any further purification.

A solution of 1.89 g (0.02 mol) of methyl chloroformate in 10 ml of toluene, was dropwise added to a mixture comprising 3.04 g (0.02 mol) of 5-methyl-2-methoxyphenylhydrazine, 3.04 g (0.022 mol) of potassium carbonate, 50 ml of toluene and 30 ml of water, under cooling with ice. The mixture was left to stand overnight at room temperature, and then subjected to liquid separation. The organic layer thereby obtained was washed with water, and then dried over anhydrous sodium sulfate. The solvent was distilled off, and the oily substance thereby obtained was purified by silica gel column chromatography, whereby 3.1 g of methyl 3-(5-methyl-2-methoxyphenyl)carbazinate was obtained.

Methyl 3-(5-methyl-2-methoxyphenyl)carbazinate was added to an excess phosgene/toluene solution, and the mixture was stirred at room temperature and left overnight. Then, the solvent was distilled off, whereby an oily substance of methyl 3-(chlorocarbonyl)-3-(5-methyl-2-methoxyphenyl)-carbazinate was obtained. The obtained oily substance was dissolved in dichloromethane, and after an addition of triethylamine, the mixture was stirred at room temperature overnight. Then, the reaction mixture was poured into ice water and extracted with dichloromethane. The organic layer thereby obtained was washed with 2N hydrochloric acid and then with a

saturated sodium chloride aqueous solution, and dried over anhydrous sodium sulfate, and then the solvent was distilled off. The product thereby obtained was purified by silica gel column chromatography, whereby Compound No. 1 as identified in Table 1 was obtained.

In the same manner as above, various compounds as identified in Table 1 were prepared from various starting materials.

Table 1

| Compound No. | 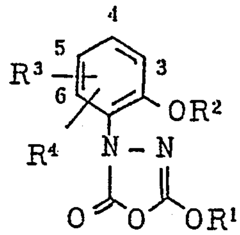 | | | | |
|---|---|---|---|---|---|
| | $R^1$ | $R^2$ | $R^3$ | $R^4$ | Physical properties |
| 1 | $CH_3$ | $CH_3$ | 5-$CH_3$ | H | m. p. 91~2° |
| 2 | 〃 | iso-$C_3H_7$ | 〃 | H | m. p. 65° |
| 3 | 〃 | $CH_3$ | 5-i-$C_3H_7$ | H | $n_D^{25}$ 1.5260 |
| 4 | 〃 | 〃 | 5-sec$C_4H_9$ | H | m. p. 48~9° |
| 5 | 〃 | 〃 | 5-t-$C_4H_9$ | H | m. p. 82~3° |
| 6 | $C_2H_5$ | 〃 | 〃 | H | m. p. 111~111.5° |
| 7 | 〃 | 〃 | 5-sec$C_4H_9$ | H | m. p. 67° |
| 8 | $CH_3$ | iso-$C_3H_7$ | 5-sec$C_4H_9$ | H | $n_D^{25.5}$ 1.5112 |
| 9 | 〃 | 〃 | 5-t-$C_4H_9$ | H | $n_D^{25.5}$ 1.5101 |
| 10 | $C_2H_5$ | 〃 | 〃 | H | $n_D^{25.5}$ 1.5062 |
| 11 | $CH_3$ | $C_2H_5$ | 5-$CH_3$ | H | $n_D^{25.5}$ 1.5231 |
| 12 | 〃 | sec-$C_5H_{11}$ | 5-$CH_3$ | H | $n_D^{25}$ 1.5124 |
| 13 | 〃 | iso-$C_4H_9$ | 5-$CH_3$ | H | $n_D^{26.5}$ 1.5130 |
| 14 | 〃 | $CH_3$ | 5-$C_2H_5$ | H | m. p. 46~7° |
| 15 | $C_2H_5$ | iso-$C_3H_7$ | 5-$CH_3$ | H | $n_D^{25}$ 1.5102 |
| 16 | $CH_3$ | 〃 | 5-$C_2H_5$ | H | $n_D^{25.5}$ 1.5152 |
| 17 | $CH_3$ | $-CH_2CH=CH_2$ | 5-$CH_3$ | H | $n_D^{25.5}$ 1.5345 |
| 18 | 〃 | iso-$C_3H_7$ | 5-iso$C_3H_7$ | H | $n_D^{25}$ 1.5120 |

| Compound No. | $R^1$ | $R^2$ | $R^3$ | $R^4$ | Physical properties |
|---|---|---|---|---|---|
| 19 | $CH_3$ | iso-$C_3H_7$ | 4-$CF_3$ | H | $n_D^{25}$ 1.4832 |
| 20 | 〃 | 〃 | 5-$CF_3$ | H | $n_D^{26.5}$ 1.4805 |
| 21 | 〃 | $CH_3$ | 〃 | H | m.p. 81~2° |
| 22 | 〃 | sec-$C_4H_9$ | 5-$CH_3$ | H | $n_D^{25.0}$ 1.5160 |
| 23 | 〃 | $C_2H_5$ | 5-iso$C_3H_7$ | H | $n_D^{25.0}$ 1.5179 |
| 24 | 〃 | $-CH_2C\equiv CH$ | 5-$CH_3$ | H | m. p. 70.5~71.0 |
| 25 | $CH_3$ | $C_2H_5$ | 4-$CH_3$ | H | m. p. 91~2° |
| 26 | $C_2H_5$ | $C_2H_5$ | 〃 | H | m. p. 85~6° |
| 27 | $CH_3$ | iso-$C_3H_7$ | 〃 | H | m. p. 72~3° |
| 28 | $C_2H_5$ | iso-$C_3H_7$ | 〃 | H | m. p. 59~60° |
| 29 | $CH_3$ | $CH_3$ | 6-$CH_3$ | H | m. p. 95~95.5° |
| 30 | $CH_3$ | iso-$C_3H_7$ | 4-$CH_3$ | 5-$CH_3$ | $n_D^{25}$ 1.5199 |
| 31 | 〃 | 〃 | 3-$CH_3$ | 5-$CH_3$ | $n_D^{25}$ 1.5144 |
| 32 | 〃 | $CH_3$ | 4-$CH_3$ | H | m. p. 95° |
| 33 | 〃 | $CH_3$ | 3-$CH_3$ | 5-$CH_3$ | m. p. 94.5~95.5° |
| 34 | 〃 | $CH_3$ | 4-$CH_3$ | 5-$CH_3$ | m. p. 114.5~115.5 |
| 35 | 〃 | iso-$C_3H_7$ | 3-iso$C_4H_9$ | H | $n_D^{25}$ 1.5048 |
| 36 | 〃 | $CH_3$ | 3-iso$C_3H_7$ | H | m. p. 79~78° |
| 37 | 〃 | iso-$C_3H_7$ | 3-iso-$C_3H_7$ | H | m. p. 103~104° |
| 38 | 〃 | $CH_3$ | 3-$CH_3$ | H | m. p. 91~92° |
| 39 | 〃 | iso-$C_3H_7$ | 3-$CH_3$ | H | m. p. 66~67° |
| 40 | 〃 | n-$C_3H_7$ | 4-$CH_3$ | H | m. p. 72~3° |

0143440

| Compound No. | $R^1$ | $R^2$ | $R^3$ | $R^4$ | Physical properties |
|---|---|---|---|---|---|
| 41 | $CH_3$ | $n-C_3H_7$ | $5-CH_3$ | H | m. p. 79~80° |
| 42 | 〃 | $C_2H_5$ | $5-C_2H_5$ | H | m. p. 50~51 |
| 43 | 〃 | $CH_3$ | $4-C_2H_5$ | H | $n_D^{24.5}$ 1.5321 |
| 44 | 〃 | $C_2H_5$ | $4-C_2H_5$ | H | m. p. 75° |
| 45 | 〃 | $iso-C_3H_7$ | $4-C_2H_5$ | H | $n_D^{24.5}$ 1.5149 |
| 46 | $C_2H_5$ | $n-C_3H_7$ | $4-CH_3$ | H | m. p. 41~2° |
| 47 | 〃 | $n-C_3H_7$ | $5-CH_3$ | H | $n_D^{24.5}$ 1.5120 |

In the following Examples for various formulations, "parts" means "parts by weight".

Wettable powder

50 parts of a compound identified in Table 1, 15 parts of clay, Carplex #80 (trademark of Shionogi & Co., Ltd.), 30 parts of N,N-Kaolin Clay (trademark of Tsuchiya Kaolin Co., Ltd.) and 5 parts of a higher alcohol sulfuric acid ester surfactant, Sorpol 8070 (trademark of Toho Chemical Industry Co., Ltd.) were blended and uniformly mixed and pulverized to obtain a wettable powder containing 50% of the active ingredient.

Granule

5 parts of a compound identified in Table 1, 38 parts of clay, 55 parts of bentonite, and 2 parts of a succinate surfactant, Air Roll CT-1 (trademark of Toho Chemical Industry Co., Ltd.) were blended and, after an addition of water, kneaded and further granulated by means of a granulating machine. Then, the product was dried at 60°C for 2 hours to obtain granules containing 5% of the active ingredient.

Emulsion

30 parts of a compound identified in Table 1 was dissolved in a solvent mixture comprising 30 parts of xylene and 25 parts of dimethylformamide, and 15 parts of a polyoxyethylene surfactant, Sorpol 3005X (trademark of Toho Chemical Industry Co., Ltd.) was further added to

obtain an emulsion containing 30% of the active ingredient.

Dust

0.5 part of a compound identified in Table 1 and 99.5 parts of clay were mixed and pulverized to obtain a dust containing 0.5% of the active ingredient.

Test Example 1

Insecticidal tests against brown planthoppers and green rice leafhoppers

12.5 mg of a compound as identified in Table 2 was dissolved in 5 ml of acetone and further diluted with 45 ml of an aqueous solution containing 200 ppm of Sorpol 3005X to prepare an insecticidal solution with a concentration of the compound being 250 ppm.

Five larvae of each of brown planthopper and green rice leafhopper in the middle instar, were released on paddy field rice seedlings set in a glass cylinder having a diameter of 3 cm and a length of 13 cm, and the upper end of the cylinder was covered with a saran net. To the test larvae and rice seedlings in the glass cylinder, 0.5 ml of the insecticidal solution having a concentration of 250 ppm was applied by means of a small spray, and after drying it in air at room temperature, they were kept at 25$^{\circ}$C in an artificially illuminated chamber.

Upon expiry of 24 hours after the treatment with the insecticidal solution, the survival and death of the test larvae were investigated, and the mortality rates were calculated. The results thereby obtained are shown in Table 2. The compound numbers in Table 2 correspond to the compound numbers in Table 1.

Table 2

| Compound No. | Mortality rate (%) | |
|---|---|---|
| | Brown planthopper | Green rice leafhopper * |
| 1 | 100 | 100 |
| 2 | 80 | 100 |
| 3 | 100 | 100 |
| 4 | 90 | 100 |
| 5 | 100 | 100 |
| 10 | 85 | 100 |
| 14 | 95 | 100 |
| 16 | 100 | 100 |
| 17 | 100 | 100 |
| 18 | 100 | 100 |
| 19 | 85 | 100 |
| 20 | 80 | 100 |
| 22 | 100 | 100 |
| 23 | 100 | 100 |
| 26 | 95 | 100 |
| 27 | 100 | 100 |
| 28 | 100 | 100 |
| 29 | 85 | 100 |

| Compound No. | Mortality rate (%) | |
|---|---|---|
| | Brown planthopper | Green rice leafhopper * |
| 30 | 85 | 100 |
| 31 | 80 | 100 |
| 32 | 90 | 100 |
| 33 | 80 | 100 |
| 34 | 75 | 100 |
| 35 | 100 | 100 |
| 36 | 90 | 100 |
| 37 | 70 | 100 |
| 38 | 100 | 100 |
| 39 | 100 | 100 |
| 40 | 100 | 100 |
| 41 | 80 | 100 |
| 42 | 100 | 100 |
| 43 | 100 | 100 |
| 44 | 100 | 100 |
| 45 | 100 | 100 |
| 46 | 100 | 100 |
| 47 | 100 | 100 |

*: Resistant strain collected at Nakagawara region in Ehime Ken, Japan

0143440

Test Example 2

Wettable powders of the compounds of the present invention and the comparative compounds as identified in Table 3 were, respectively, diluted with water (to a concentration of 50 ppm), and then applied in the same manner as in Test Example 1. Twenty four hours later, the mortality rates of brown planthoppers were investigated. The tests were repeated 4 times, and average mortality rates were obtained. The results are shown in Table 3.

Table 3

| Compound No. (50ppm) | Mortality rate (%) |
|---|---|
| 1 | 60 |
| 3 | 80 |
| 4 | 60 |
| 5 | 85 |
| 6 | 65 |
| 8 | 90 |
| 11 | 100 |
| 13 | 60 |
| 14 | 80 |
| 15 | 90 |
| 16 | 95 |
| 17 | 100 |
| 18 | 100 |
| 22 | 80 |
| 23 | 90 |

| Compound No. (50ppm) | Mortality rate (%) |
|---|---|
| 26 | 70 |
| 33 | 70 |
| 38 | 80 |
| 39 | 90 |
| 40 | 100 |
| 41 | 70 |
| 43 | 100 |
| 44 | 80 |
| 45 | 100 |
| 46 | 90 |
| Comparative Compounds | |
| * 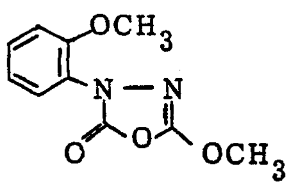 | 30 |
| ** 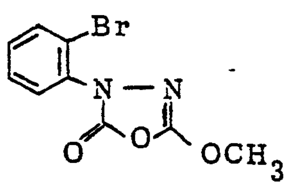 | 30 |
| ** 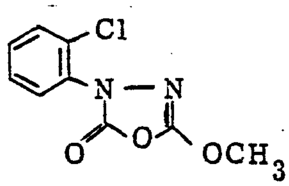 | 40 |

* : The compound disclosed in Japanese Unexamined Patent Publication No. 101981/1976

** : The compound disclosed in German Patent No. 2604110

Test Example 3

The insecticidal activities were tested by using dusts containing 0.5% by weight of the respective active ingredients.

Ten green rice leafhoppers having resistance against insecticides and paddy field rice seedlings were put in a cylinder of a metal net (20 mesh) having a diameter of 3 cm and a height of 20 cm. 200 mg of each dust was applied from outside the metal net by means of a Belger duster (corresponding to a dose of 4 kg/10 a). Twenty four hours later, the death and survival were investigated, and the mortality rates were calculated. The above test was repeated 4 times for each compound, and average mortality rates were calculated. The results are shown in Table 4.

Table 4

| Compound No. | Mortality rate (%) |
|---|---|
| 1 | 100 |
| 2 | 90 |
| 3 | 88 |
| 5 | 80 |
| 8 | 76 |
| 9 | 92 |

| Compound No. | Mortality rate (%) |
|---|---|
| 10 | 90 |
| 13 | 85 |
| 14 | 100 |
| 15 | 100 |
| 16 | 100 |
| 17 | 100 |
| 22 | 100 |
| 23 | 100 |
| 25 | 100 |
| 26 | 100 |
| 27 | 100 |
| 28 | 100 |
| 30 | 100 |
| 32 | 95 |
| 33 | 89 |
| Comparative Compounds | |
| 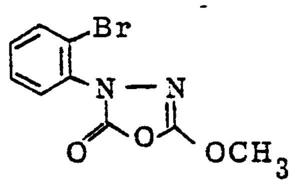 | 0 |
| 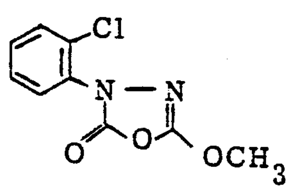 | 0 |

Test Example 4

In the same manner as in Test Example 3, the insecticidal activities against brown planthoppers were investigated with respect to the compounds of the present invention and the comparative compounds by using dusts containing 0.5% of the respective active ingredients. The results are shown in Table 5.

Table 5

| Compound No. | Mortality rate (%) |
|---|---|
| 1 | 90 |
| 3 | 95 |
| 5 | 84 |
| 11 | 97 |
| 13 | 75 |
| 14 | 78 |
| 15 | 80 |
| 16 | 95 |
| 17 | 95 |
| 18 | 80 |
| 19 | 80 |
| 22 | 100 |
| 23 | 95 |
| 24 | 89 |
| 25 | 100 |
| 26 | 95 |
| 27 | 100 |
| 28 | 100 |
| 31 | 100 |

| Compound No. | Mortality rate (%) |
|---|---|
| Comparative Compound 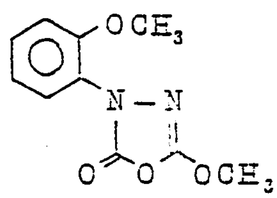 | 35 |

Test Example 5

Insecticidal tests against green peach aphids

Wettable powders of the compounds of the present invention were diluted with water to a predetermined concentration (50 ppm). In each solution thus prepared, a raddish seedling infested with green peach aphids (*Myzus percicae* Sulzer) was dipped for a few seconds, then dried in air at room temperature and kept in an artificially illuminated chamber at 25°C. Twenty four hours after the treatment with the solution, the death and survival were investigated, and the controlling value was calculated in accordance with the following equation, in comparison with the number of parasites counted prior to the treatment with the insecticidal solution. The results are shown in Table 6.

$$\text{Controlling value (\%)} = \frac{\text{Mortality rate in the treated area} - \text{Mortality rate in the non-treated area}}{100 - \text{Mortality rate in the non-treated area}} \times 100$$

Table 6

| Compound No. | Controlling value (%) |
| --- | --- |
| 1 | 88 |
| 2 | 100 |
| 3 | 100 |
| 4 | 100 |
| 5 | 100 |
| 6 | 88 |
| 7 | 100 |
| 14 | 97 |
| 20 | 100 |
| 23 | 100 |
| 24 | 97 |
| 25 | 100 |
| 26 | 100 |
| 32 | 72 |
| 33 | 77 |
| 34 | 85 |
| Comparative Compounds | |
| 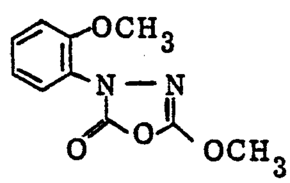 | 35 |
| 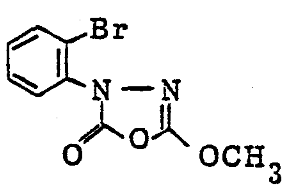 | 0 |
| 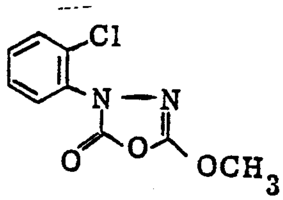 | 0 |

Test Example 6

In order to investigate the synergistic effect of a combination of the insecticide of the present invention with a carbamate, insecticidal compositions having various proportions of (a) Compound No. 32 in Table 1 and (b) m-tolyl-N-methylcarbamate (MTMC), were prepared. The $LD_{50}$ values with respect to green rice leafhoppers were determined in accordance with the following method, and the synergistic coefficients were calculated.

The test compounds were dissolved in acetone to obtain acetone solutions having various concentrations. Each solution was injected to the chest of a female adult of green rice leafhopper in an amount of 0.2 $\mu\ell$ per adult.

The treated insects were put in a plastic cup and kept in a constant temperature chamber at $26 \pm 1^{\circ}C$. Twenty four hours later, the death and survival were investigated. 20 female adults of green rice leafhopper were used for a test of each compound.

The synergistic coefficients were calculated by the following equation in accordance with Sun and Johnson [J. Econ. Entomol. 53, 887 (1960)]. When the value is 100 or more, the synergistic effect is present.

$$\text{Synergistic coefficient K} = \frac{LD_{50} \text{ of Compound No. 32}}{LD_{50} \text{ of the mixed composition}} \times 100$$

Table 7

| Test compound a b | $LD_{50}$ (µg/g) | Synergistic coefficient K |
|---|---|---|
| 1 : 0 | 4.0 | |
| 0 : 1 | 71 | |
| 1 : 1 | 5.7 | 140 |
| 1 : 2 | 5.9 | 200 |
| 1 : 3 | 7.9 | 200 |
| 1 : 4 | 8.5 | 240 |
| 1 : 5 | 7.3 | 330 |
| 1 : 10 | 8.9 | 490 |
| 1 : 20 | 10.8 | 780 |
| 1 : 30 | 10.3 | 1210 |

Test Example 7

Dusts containing Compound No. 32 and MTMC as active ingredients in the respective amounts as identified in Table 8, were prepared. Insecticidal tests against green rice leafhoppers were conducted in the same manner as in Test Example 3. The results are shown in Table 8. Ten green rice leafhoppers were tested for each test, and average values of four tests are shown.

Table 8

| Compound (parts by weight) No. 32 | MTMC | Mortality rate (%) 3 hrs later | 24 hrs later |
|---|---|---|---|
| 1 | 1 | 90.0 | 100 |
| 0.5 | 1.5 | 95.0 | 100 |
| 0.33 | 1.67 | 82.5 | 95 |
| 0.18 | 1.82 | 32.5 | 80.0 |
| 0.5 | 0 | 12.5 | 55.0 |
| 0.33 | 0 | 5.0 | 20.0 |
| 0 | 2.0 | 0 | 7.5 |
| 0 | 0 | 0 | 5.0 |

CLAIMS:

1. An insecticidal composition comprising an insecticidally effective amount of at least one oxadiazolinone derivative represented by the general formula:

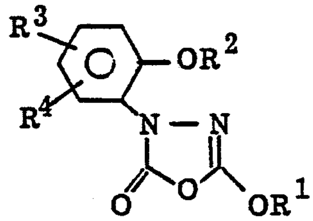

(I)

where $R^1$ is an alkyl group having from 1 to 5 carbon atoms, $R^2$ is an alkyl group having from 1 to 5 carbon atoms, and an alkenyl group having from 2 to 5 carbon atoms or an alkynyl group having from 2 to 5 carbon atoms, and each of $R^3$ and $R^4$ is a hydrogen atom, an alkyl group having from 1 to 4 carbon atoms or a trifluoromethyl group, provided that $R^3$ and $R^4$ are not simultaneously hydrogen atoms, and an adjuvant.

2. The insecticidal composition according to Claim 1, wherein $R^1$ in the formula I is an alkyl group having 1 or 2 carbon atoms.

3. The insecticidal composition according to Claim 2, wherein $R^2$ in the formula I is an alkyl group having 1 or 2 carbon atoms.

4. The insecticidal composition according to Claim 3, wherein either one of $R^3$ and $R^4$ in the formula I is a hydrogen atom, and the other is a methyl group.

5. The insecticidal composition according to Claim 4, wherein either one of $R^3$ and $R^4$ in the formula I is a methyl group attached to the 4- or 5-position of the phenyl group.

6. The insecticidal composition according to Claim 1, wherein the compound of the formula I is selected from the group consisting of 3-(2-ethoxy-5-methylphenyl)-5-methoxy-1,3,4-oxadiazoline-2-one, 3-(2-ethoxy-4-methylphenyl)-5-methoxy-1,3,4-oxadiazoline-2-one and 3-(2-methoxy-4-methylphenyl)-5-methoxy-1,3,4-oxadiazoline-2-one.

7. The insecticidal composition according to Claim 1, which further contains a carbamate insecticide.